# EUROPEAN PATENT APPLICATION

(11) **EP 4 005 639 A1**
(43) Date of publication of application: **01.06.2022**
(21) Application number: 20846022.0
(22) Date of filing: 22.07.2020
(51) Int. Cl.: A61P 27/02, A61P 29/00, A61P 35/00, A61K 9/00, A61K 9/48, A61K 47/02, A61K 47/58, A61K 47/59, A61K 47/60, A61M 37/00

(54) **CONTINUAL DRUG RELEASE DEVICE THAT CAN BE REPLENISHED WITH DRUGS**

(30) Priority: 26.07.2019 JP 2019138248
(71) Applicant: Tohoku University, Sendai-shi, Miyagi 980-8577 (JP)
(72) Inventor: KAJI Hirokazu, Sendai-shi, Miyagi 980-8577 (JP); ITO Yuya, Sendai-shi, Miyagi 980-8577 (JP); NAGAI Nobuhiro, Sendai-shi, Miyagi 980-8577 (JP); ABE Toshiaki, Sendai-shi, Miyagi 980-8577 (JP)
(74) Representative: Clarenbach, Carl-Philipp
(86) International application number: PCT/JP2020/028357
(87) International publication number: WO 2021/020252

(57) **Abstract**

An embeddable and replenishable controlled drug release device which achieves drug delivery in which the drug release rate can be controlled is provided. A controlled drug release device for implantation in a body that can be replenished with a drug using a syringe and a needle in the state of being implanted in the body, wherein the device has a capsule structure and includes (i) a controlled drug release part for controlled-release of a drug from the device, (ii) a drug storage part for keeping the drug and (iii) a drug introduction part for introducing the drug into the drug storage part, wherein the controlled drug release part has a controlled-release face having a pore, a hydrogel is retained in the controlled drug release part, the drug storage part is adjacent to the controlled drug release part, the drug kept in the drug storage part is released from the pore in the controlled drug release part through the controlled drug release part, and the drug is introduced into the drug storage part through the drug introduction part using a syringe and a needle.

## Description

### Technical Field

The present invention relates to the field of drug delivery devices. Specifically, the invention relates to a controlled drug release device which can be embedded in a living body, and further specifically, the invention relates to a device which achieves controlled-release over a long period at a constant sustained rate and which can be replenished with a drug.

### Background Art

An important issue in drug therapy is to deliver a necessary amount of a drug to the target site of the therapy when needed. This is studied widely as a drug delivery system (drug delivery, DDS). For example, instillation is a general administration method for drug administration for eye diseases, but while instillation is effective for diseases in the anterior eye segment, therapeutic effects on diseases in the posterior eye segment are difficult to obtain because a sufficient amount of the drug does not always move to the posterior eye segment due to the corneal barrier, the clearance by the lacrimal fluid or the like. Accordingly, intravitreal injection is used in the treatment of age-related macular degeneration, which is an intractable disease in the posterior eye segment (retinal disease). Although the therapeutic effect of inhibiting retinal degeneration is obtained, there are problems of not a low risk of intraocular side effects due to the necessity for frequent administration of around once a month and the high medical costs. For the treatment of retinitis pigmentosa, which is an intractable retinal disease, it is reported that isopropyl unoprostone exhibits the effect of inhibiting retinal degeneration, and a therapeutic method using a sustained transscleral administration device is examined (NPL 1). The transscleral administration method is expected to be a safe administration method which is not invasive at all to the intraocular part because a controlled drug release device is simply placed on the eyeball (on the sclera). However, when the drug in the device is used up, a surgery is required to make an incision in the conjunctiva, as in embedding, remove the device from the sclera and embed a new device again. If the embedded device can be refilled with the drug, the removal will not be necessary, and sustained treatment may be continued through refilling treatment which can be conducted through treatment as an outpatient.

It is reported that an injectable gel is used for producing a device that can be replenished with a drug (see PTL 1). However, the production of an injectable gel requires complicated operations, and a method for more simply injecting a liquid drug into the device is required considering the clinical applications.

### Citation List

### Patent Literature

PTL 1: International Publication WO2018/143481

### Non Patent Literature

NPL 1: Adv Healthc Mater. 2014 Oct;3(10): 1555-60

### Summary of Invention

### Technical Problem

Although a replenishable-type controlled drug release device has been developed, the method for use is complicated. Considering the actual use, because the administration period, the dosage and the administration site vary with the disease and the individual, a device which can control the release in an order-made manner for a long period and closely fits the shape of the aimed site and which can be produced without the need of complicated operations is useful.

An object of the invention is to provide an embeddable and replenishable controlled drug release device which achieves drug delivery that can control the drug release rate at a desired release rate over a long period of several months or longer and can be produced without complicated operations and into which a liquid drug can be injected simply.

### Solution to Problem

To solve the problem of the removal of the device after the drug release that the conventional transscleral DDS devices have, reinjection of a drug into the device at the site is required. Use of an injectable gel is reported as a solution thereof. However, the production of an injectable gel requires complicated operations, and a method for more simply injecting a liquid drug into the device has been desired considering the clinical applications.

The present inventors have considered to place a hydrogel such as a polyvinyl alcohol gel which is not easily decomposed in advance in the device and to use the gel. The gel is cured in the device in advance, and the drug solution is then injected for drug release. The reinjection of the drug is achieved thereafter. After an *in vitro* drug release test using gels, use of the selected hydrogel in the device was assessed, and the invention has been then completed.

That is, the invention is as follows.
[1] A controlled drug release device for implantation in a body that can be replenished with a drug using a syringe and a needle in the state of being implanted in the body,
   wherein the device has a capsule structure and includes
      (i) a controlled drug release part for controlled-release of a drug from the device,
      (ii) a drug storage part for keeping the drug, and
      (iii) a drug introduction part for introducing the drug into the drug storage part,
   wherein the controlled drug release part has a controlled-release face having a pore,
   a hydrogel is retained in the controlled drug release part,
   the drug storage part is adjacent to the controlled drug release part,
   the drug kept in the drug storage part is released from the pore in the controlled drug release part through the controlled drug release part, and
   the drug is introduced into the drug storage part through the drug introduction part using a syringe and a needle.
[2] The controlled drug release device that can be replenished with a drug of [1], wherein the device having a capsule structure is made of polydimethylsiloxane (PDMS).
[3] The controlled drug release device that can be replenished with a drug of [1], wherein the device having a capsule structure is made of triethylene glycol dimethacrylate (TEGDM) and/or polyethylene glycol dimethacrylate (PEGDM) and has a layer of polydimethylsiloxane (PDMS) or urethane in the drug introduction part.
[4] The controlled drug release device that can be replenished with a drug of [1], wherein the device having a capsule structure is made of triethylene glycol diacrylate (TEGDA) and/or polyethylene glycol diacrylate (PEGDA) and has a layer of polydimethylsiloxane (PDMS) or urethane in the drug introduction part.
[5] The controlled drug release device that can be replenished with a drug of [1] or [2], wherein the hydrogel retained in the controlled drug release part is an agarose gel (AGA gel), a polyvinyl alcohol gel (PVA gel), a polyacrylamide gel (AAm gel) or a double network gel (DN gel).
[6] The controlled drug release device that can be replenished with a drug of any of [1] to [4], wherein the hydrogel retained in the controlled drug release part is a polyvinyl alcohol gel (PVA gel), a mixture of polyethylene glycol dimethacrylate (PEGDM) and triethylene glycol dimethacrylate (TEGDM), a mixture of polyethylene glycol diacrylate (PEGDA) and triethylene glycol diacrylate (TEGDA) or polydimethylsiloxane having a porous structure (porous PDMS).
[7] The controlled drug release device that can be replenished with a drug of any of [1] to [6] which can keep a liquid as the drug in the drug storage part.
[8] The controlled drug release device that can be replenished with a drug of any of [1] to [7] which has an incorporated metal wire for changing and stabilizing the shape.
[9] The controlled drug release device that can be replenished with a drug of any of [1] to [8] which is a scleral placement device for implantation on a sclera.
[10] The controlled drug release device that can be replenished with a drug of any of [1] to [8] which is a subcutaneously embedded device for subcutaneous embedding.

The present specification includes the contents disclosed in Japanese patent application No. 2019-138248, which is the basis for the priority claim of the present application.

### Advantageous Effects of Invention

When a controlled drug release device produced by introducing a hydrogel such as a polyvinyl alcohol gel into the inside is used, linear controlled-release of the drug is observed even after repeated reinjection.

### Brief Description of Drawings

[Fig. 1] A figure illustrating a mechanism for drug release/drug injection using a PVA gel.
[Fig. 2] A figure illustrating a mechanism for drug release/drug injection using a PVA gel (partial introduction of a PVA gel).
[Fig. 3] A figure showing the results of an *in vitro* drug release test using four kinds of gel.
[Fig. 4] A figure showing a SEM image of a PVA gel.
[Fig. 5] A figure showing the change of the device with time in an *in vitro* drug release test.
[Fig. 6] A figure showing the results of an *in vitro* drug release test using PDMS capsule-PVA gel.
[Fig. 7] A figure showing the state of PDMS capsule-PVA gel after reinjection of an aqueous FL solution.
[Fig. 8] A figure showing the results of an *in vitro* reinjected drug release test using PDMS capsule-PVA gel.
[Fig. 9] A figure showing the results of an *in vitro* drug release test using PDMS capsule-partial PVA gel.
[Fig. 10] A figure illustrating the structure of a controlled drug release device having a tubular shape (Embodiments 2 and 3).
[Fig. 11] The controlled drug release part and the drug introduction part of a controlled drug release device having a tubular shape are shown.
[Fig. 12] A figure showing controlled-release of a drug from a tubular controlled drug release device produced with a 3D printer.
[Fig. 13] Fig. 13A shows a produced device for rabbit containing FITC-ALB, and Fig. 13B is a figure showing the integrated amount of FITC-ALB released into DPBS.
[Fig. 14] Pictures of the eyeballs and the devices taken out on the second week after implanting the devices (each N=4).
[Fig. 15] A figure showing the tissue FITC-ALB concentrations (N=4) in the retina, the choroid/RPE, the sclera, the iris, the aqueous humor and the vitreous fractioned from the eyeballs taken out on the second week after implanting the devices.

### Description of Embodiments

The invention is explained in detail below.

The invention is a continual drug release device for implantation in a body which contains a drug in the inside and which can conduct controlled-release of the drug, and the invention is a replenishable continual drug release device which can be replenished with a drug, that is, the device can be refilled. Moreover, the device of the invention is also a drug delivery device for delivering a drug.

### 1. Structure and Materials of Controlled Drug Release Device of Invention

The continual drug release device that can be replenished with a drug has a capsule structure. The capsule structure here means a structure which can contain a drug or the like in the inside, namely a structure having space which functions as a reservoir for containing a substance in the inside. The capsule structure is, for example, a capsule structure composed of a box-type reservoir which is closed with a sheet-shaped cover. The inside of the capsule can be filled with a drug. The box-type here means a shape which can contain a substance in the inside. A capsule having no cover is sometimes called a reservoir. The structure of the controlled drug release device of the invention can also be called a tubular capsule structure having pores at the front end and the rear end.

The controlled drug release device of the invention contains a hydrogel which is not easily decomposed in the state of being retained in the device and releases a drug using the hydrogel.

The controlled drug release device of the invention is composed of a controlled drug release part, a drug storage part and a drug introduction part.

The controlled drug release part contains the hydrogel in the inside, has a controlled-release face and releases a drug from the controlled-release face.

The drug storage part contains a liquid drug in the inside.

The drug introduction part is a part adjacent to the drug storage part, and the drug is injected into the drug storage part through the drug introduction part with a syringe or the like.

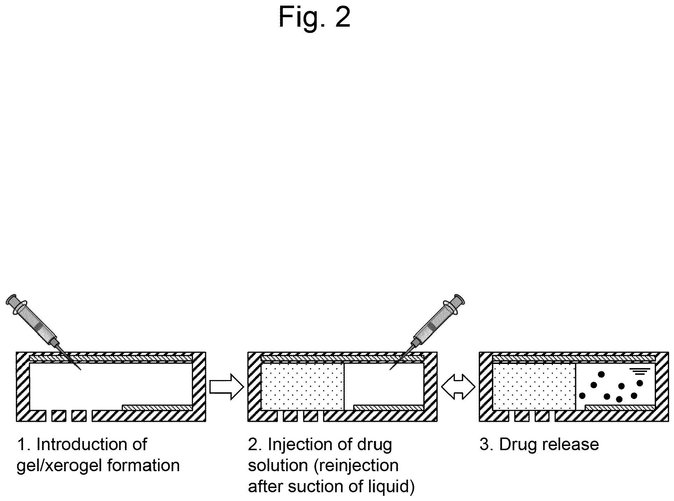

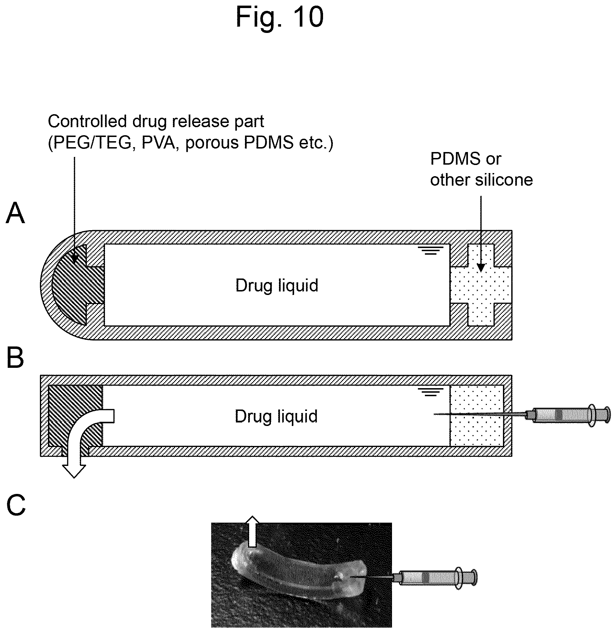

Examples of the controlled drug release device of the invention are illustrated in Fig. 2 and Fig. 10. Here, the controlled drug release device illustrated in Fig. 2 is called a controlled drug release device of Embodiment 1, and the controlled drug release device illustrated in Fig. 10 is called a controlled drug release device of Embodiment 2. A device using a tubular TEGDM capsule formed with a 3D printer is called a controlled drug release device of Embodiment 3.

The controlled drug release device of the invention has a capsule structure and has a controlled-release face on a part of the faces constituting the capsule structure. The controlled-release face has pores for letting the drug pass through. The pores may be provided in only particular one face of the device or provided in multiple faces. The pores may be provided in the entire face(s) or provided in a part of the face(s). Because the drug is released from a face having the pores, the face having the pores is called a controlled-release face. The pore size is 0.001 µm to 10 mm, and the pore density is 1 to 2000 pore/cm².

A hydrogel is introduced into the controlled drug release part and is cured. Here, the hydrogel is introduced into a part of the controlled drug release device having the controlled-release face and is cured. In an actual case, the part having the cured hydrogel is the controlled drug release part. The controlled drug release part accounts for 10 to 80%, preferably 25 to 75%, further preferably 40 to 60%, further preferably 50% of the capacity of the internal space of the controlled drug release device. The controlled drug release part is adjacent to the drug storage part, and the controlled drug release part and the drug storage part can be distinguished from each other by the presence or the absence of the hydrogel. A liquid drug, namely a solution of a drug, is injected into the drug storage part. The part of the device having a capsule structure which is adjacent to the drug storage part is the drug introduction part. The hydrogel that has been introduced into the controlled drug release part and cured is illustrated in Fig. 2, middle.

As the hydrogel, a gel which is a biocompatible material and which is not easily decomposed is used, and an agarose gel (AGA gel), a polyvinyl alcohol gel (PVA gel), a polyacrylamide gel (AAm gel) or a double network gel (DN gel) can be used. The agarose gel contains 1 to 5 wt%, preferably 1 to 3 wt%, further preferably 2 wt% agarose. The polyvinyl alcohol gel contains 5 to 30 wt%, preferably 10 to 20 wt%, further preferably 15 wt% polyvinyl alcohol. The polyacrylamide gel is a gel obtained by mixing acrylamide and methylenebisacrylamide and is obtained by mixing and polymerizing 100 to 200 mg/mL, preferably 150 to 210 mg/mL, further preferably 180 mg/mL of acrylamide and 0.05 to 0.25 mg/mL, preferably 0.08 to 0.15 mg/mL, further preferably 0.118 mg/mL of methylenebisacrylamide. For the polymerization, several microliters per 10 mL to several dozen microliters per 10 mL of a polymerization agent such as APS (ammonium persulfate) and several dozen microliters per 10 mL to several hundred microliters per 10 mL of a polymerization accelerator such as TEMED (tetramethylethylenediamine) may be added. The double network gel refers to an ultra-high-strength gel which is obtained by combining two different elements and which contains about 90% water content. A known double network gel can be used, but a DN gel can be obtained, for example, by UV-crosslinking of a polymerization solution of acrylamide methylpropane sulfonic acid and methylenebisacrylamide and immersion of the obtained gel in a polymerization solution of acrylamide and methylenebisacrylamide for further UV-crosslinking.

Furthermore, the hydrogel can be a dimethacrylate (DM) mixture, a diacrylate (DA) mixture or polydimethylsiloxane having a porous structure (porous PDMS). Polyethylene glycol dimethacrylate (PEGDM) and triethylene glycol dimethacrylate (TEGDM) are used for the dimethacrylate (DM) mixture, and polyethylene glycol diacrylate (PEGDA) and triethylene glycol diacrylate (TEGDA) are used for the diacrylate (DA) mixture. The mixture of PEGDM and TEGDM is a mixture of 30 to 70% (w/w) PEGDM and 70 to 30% (w/w) TEGDM, preferably a mixture of 40% (w/w) PEGDM and 60% (w/w) TEGDM. The mixture of PEGDA and TEGDA is a mixture of 30 to 70% (w/w) PEGDA and 70 to 30% (w/w) TEGDA, preferably a mixture of 40% (w/w) PEGDA and 60% (w/w) TEGDA. PEGDM or PEGDA can control the release property when mixed with water. The mixture of PEGDM or PEGDA and water is a mixture of 1 to 99% (w/w) PEGDM or PEGDA and 99 to 1% (w/w) water. The molecular weight of PEGDM or PEGDA is not limited, but molecular weights of 330 (4G), 536 (9G), 736 (14G), 1136 (23G) and the like can be used. The molecular weight is preferably 330 (4G), 536 (9G) or 736 (14G).

The hydrogel can be introduced into the controlled drug release part of the controlled drug release device, cured and formed into a xerogel. For example, in the case of a polyvinyl alcohol gel, an aqueous polyvinyl alcohol gel solution can be introduced into the controlled drug release part and subjected to repeated freeze-drying to fill the controlled drug release part with the polyvinyl alcohol gel, and a xerogel can be thus obtained.

When the hydrogel is retained in the controlled drug release device, the drug is not contained in the hydrogel. After introducing the drug into the drug storage part, when the drug is released from the pores in the controlled-release face of the controlled drug release part through the hydrogel in the controlled drug release part, the drug is then contained in the hydrogel in the controlled drug release part.

As the material of the continual drug release device that can be replenished with a drug of the invention, a material which does not adhere to the living body is preferably used. A material which does not adhere to the living body is a substance which is dissolved in the living body and which does not adhere or bind to the tissues of the living body.

To enable the reinjection of a drug, it is necessary that the device is flexible and plastic and that a needle used for the reinjection can be stuck and can be repeatedly stuck because the hole is filled immediately after the needle is stuck. Although a large part of the device may be made of a harder material, when a material which is so hard that a needle cannot be stuck is used, the drug introduction part into which a needle is stuck for the reinjection of the drug should be made of a material into which a needle can be stuck repeatedly.

The material of the outer part of the capsule into which a needle can be stuck repeatedly is polydimethylsiloxane (PDMS), which is a silicone-based thermosetting resin.

The hard material into which a needle can be hardly stuck is triethylene glycol dimethacrylate (TEGDM), polyethylene glycol methacrylate (PEGDM), triethylene glycol acrylate (TEGDA) or polyethylene glycol acrylate (PEGDA). When produced with TEGDM and PEGDM or TEGDA and PEGDA, PDMS and urethane, into which a needle can be stuck repeatedly, are preferable as the material of the drug introduction part.

The controlled drug release device of Embodiment 1 is a controlled drug release device in which the entire capsule structure is made of polydimethylsiloxane (PDMS).

The hydrogel retained in the controlled drug release device of Embodiment 2 is preferably an agarose gel (AGA gel), a polyvinyl alcohol gel (PVA gel), a polyacrylamide gel (AAm gel) or a double network gel (DN gel).

In the controlled drug release device of Embodiment 2, the capsule structure is made of triethylene glycol dimethacrylate (TEGDM) and/or polyethylene glycol dimethacrylate (PEGDM) or triethylene glycol diacrylate (TEGDA) and/or polyethylene glycol diacrylate (PEGDA) and has a layer of PDMS or urethane in the drug introduction part. Although the controlled drug release device of Embodiment 2 also has the drug introduction part adjacent to the drug storage part, the drug introduction part of the controlled drug release device of Embodiment 2 is made of a material into which a syringe cannot be stuck and thus includes a layer of a material into which a needle can be stuck in the inside.

The thickness of the film forming the capsule structure is 0.01 mm to 5 mm, preferably 0.2 mm to 0.6 mm.

As the hydrogel retained in the controlled drug release device of Embodiment 2, in addition to polyvinyl alcohol, as the hydrogel, a mixture of polyethylene glycol dimethacrylate (PEGDM) and triethylene glycol dimethacrylate (TEGDM), a mixture of polyethylene glycol diacrylate (PEGDA) and triethylene glycol diacrylate (TEGDA) and polydimethylsiloxane having a porous structure (porous PDMS ) are preferable.

The controlled drug release device of the invention has a controlled-release face in the controlled drug release part. The "controlled-release face of the device" refers to a face which let the drug pass through and which enables controlled-release, of the faces of the device. The controlled-release face may be one face only, or multiple controlled-release faces may be included. The controlled-release face has pores, and the drug is released through the pores.

The shape of the controlled drug release device that can be replenished with a drug has a top surface, a bottom surface and a side surface and is preferably an approximate disk in which the top surface and the bottom surface are approximate circles or an approximate cube in which the top surface and the bottom surface are approximate rectangles. As long as the shape has a face for controlled-release of a substance contained in the inside, the shape is not limited, and a shape such as a flat plate, an approximate globe, an approximate tube and an approximate cylinder may be used. In the case of an approximate disk, the diameters of the top surface and the bottom surface of the approximate circles are several dozen millimeters, preferably 5 to 500 mm, further preferably 10 to 300 mm, particularly preferably 20 to 300 mm. In the case of an approximate cube, the length of a side of the top surface and the bottom surface of the approximate rectangles is several dozen millimeters, preferably 5 to 500 mm, further preferably 10 to 300 mm, particularly preferably 20 to 300 mm. The areas of the top surface and the bottom surface are approximately about 20 mm² to 200000 mm², and the thickness is several millimeters, preferably 1 to 7 mm, further preferably 2 to 5 mm.

The sizes above are examples, and the size of the device can be appropriately designed depending on the carried drug amount based on the implantation site or the purpose of treatment.

A wire of copper or stainless steel may be incorporated into the controlled drug release device of the invention to stabilize the shape. To obtain a wire-having device, the capsule structure may be cured while a wire bent into the shape of the capsule is submerged in the container used as a mold for producing the capsule structure. When a metal wire is incorporated into the device, the shape of the device can be freely changed. Thus, the device can be shaped in accordance with a complicated shape of a living body and can be implanted in such a manner that the device is in contact with the sclera or a subcutaneous tissue without any gap.

For the controlled drug release device of Embodiment 3, a capsule formed using a 3D printer is used. As the material, TEGDM or PEGDM, which are photocurable resins, or the like can be used. The shape may be any shape as long as the device has a controlled drug release part, a drug storage part and a drug injection part. When the device is used for local administration to the posterior eye segment, an arched capsule having a curvature which fits the curved surface of the eyeball is suitably used. The device preferably has the controlled drug release part at the front end of the device (the posterior eye segment side) and the drug introduction part at the rear end of the device (the anterior eye segment side). Those described above can be used as the structures of the controlled drug release part, the drug storage part and the drug injection part. As the 3D printer, any apparatus may be used. The shape of the controlled drug release device produced with a 3D printer is not limited, but the device has, for example, a shape which is the same as or similar to that of the controlled drug release device of Embodiment 2 illustrated in Fig. 10.

### 2. Production Method of Controlled Drug Release Device of Invention

A method for producing the controlled drug release device of Embodiment 1 of the invention is explained below.

The capsule constituting the outer part is produced by feeding a thermosetting resin (a PDMS prepolymer) into a PDMS mold having the shape of the capsule and thermally curing the resin. The PDMS mold is produced by feeding a thermosetting resin (a PDMS prepolymer) onto an acrylic plate on which the device shape is cut and thermally curing the resin. The PDMS mold is subjected to oxygen plasma treatment and silanization treatment so that the cast material (PDMS) is released easily. The device shape can be cut on the acrylic plate, for example, by cutting with a CAD-CAM micromachine or by three-dimensional forming with a 3D printer.

When the controlled drug release device has a reservoir part and a sheet-shaped cover part, the cover of the box-type reservoir constituting the outer part is produced by feeding the thermosetting resin (the PDMS prepolymer) in a PDMS mold having the shape of the cover and thermally curing the resin. The PDMS mold is produced in the same manner as in above.

The replenishable controlled drug release device has pores (micropores) for controlled-release of a drug. The pores can be formed by providing needle-like protrusions on the top surface or the bottom surface or on the top surface and the bottom surface of the mold for producing the part of the controlled-release device. Alternatively, holes may be made using a needle, a laser beam or the like after producing the part of the controlled-release device or the controlled-release device. The part having the pores can be called a controlled-release membrane because the drug in the capsule is released from the pores.

The hydrogel introduced into the controlled drug release part of the controlled drug release device may be introduced and cured as described in "Structure and Materials of Controlled Drug Release Device of Invention" above.

After introducing the hydrogel into the controlled drug release part and curing the hydrogel, a solution containing a drug may be injected into the drug storage part. The drug in the solution in the drug storage part soaks into the controlled drug release part and is released from the controlled-release face of the controlled drug release part.

When the device is formed with a 3D printer, a device having a capsule structure or a device having both a reservoir and a cover is designed using CAD software. At this point, the pores for the controlled drug release part and the space for the drug introduction part are left in a part thereof. This is formed with a 3D printer of stereolithography type. The base material is TEGDM, PEGDA or the like. After the formation, the device is finished by introducing the hydrogel into the controlled drug release part and PDMS into the drug introduction part.

### 3. Use Methods of Controlled Drug Release Device of Invention

Replenishable continual drug release devices can be classified by the applications, and examples thereof include a scleral placement device for the purpose of transscleral administration of a drug into an eye through the sclera of the eye and a subcutaneously embedded device for the purpose of subcutaneous systemic administration.

The structures and the use methods of the devices are explained below.

### (1) Scleral Placement Device

A scleral placement device is a device for treatment of an eye disease and is implanted or embedded on the sclera as an implant. The sclera here means the portion above the choroid and beneath the conjunctiva and thus refers to under the sclera, in the sclera, on the sclera, under the conjunctiva and on the choroid. The implantation does not require vitreous surgery, and the device can be implanted safely and simply. The scleral placement device may be implanted to place the device on the sclera at the outer corner side of the eye. The scleral placement device may have pores only in one face of the capsule and may be implanted in a manner that the controlled-release face having the pores is in contact with the sclera. The drug released from the scleral placement device reaches the inside of the eye through the sclera without being diffused to other parts. By providing a stainless-steel sheet on the bottom of the device, an accidental puncture with a needle can be prevented, and the drug can be injected safely into the device embedded on the sclera.

### (2) Subcutaneously Embedded Device

A subcutaneously embedded device is an embedded-type device which can be embedded in a subcutaneous tissue and has space which functions as a capsule for containing a substance in the inside. The device is also called a subcutaneously implanted device. When the subcutaneously embedded device is embedded subcutaneously, subcutaneous controlled-release of a drug is enabled over a long period, and the drug can also reach the whole body through the vascular system.

When the hydrogel is cured and retained in the controlled drug release part of the controlled drug release device and when a liquid of a drug is injected into and kept in the drug storage part, which is empty space adjacent to the controlled drug release part, the drug permeates through the hydrogel, moves from the drug storage part and is released from the controlled-release face of the controlled drug release part. That is, the hydrogel in the controlled drug release part has the property of letting the drug permeate through.

The drug contained in the inside is released from the controlled-release face of the device above. When the drug inside is completely released, a liquid containing the drug can be reinjected. Reinjection may be conducted with a syringe and a needle (an injector). That is, a syringe is filled with a liquid containing the drug, and the needle of the syringe is stuck in the device to replenish the drug storage part in the device. A fine needle is suitable for puncture in the skin, but 14G to 32G needles can be used. The needle is preferably a 18G to 25G needle. Regarding the shape of the needle, a shape suitable for the implantation site may be selected. A long needle, a short needle, a 1/2 curved type, a dull needle or a Huber needle suitable for repeated puncture can be used. In the case of the controlled drug release device of Embodiment 1, the needle may be stuck in the face made of PDMS having flexibility of the faces of the device, and in the case of the controlled drug release device of Embodiment 2, the needle may be stuck in the layer of PDMS or urethane provided in the drug introduction part. Injection of the drug is sometimes inhibited because the storage part is highly sealed, and thus another needle for releasing the internal pressure during the drug injection may be stuck to leak the internal pressure. Because the device of the invention can be replenished in this manner, the drug can be continually administered, and the treatment of a disease can be continued. The reinjection of the drug can be repeated, and the drug can be released and reinjected repeatedly.

Because PDMS, TEGDM, PEGDM and the like, which react weakly with the living body, are used, the device of the invention only weakly adheres, is removed easily and can be removed without a surgery when the treatment is no longer necessary or when side effects are caused. That is, the device of the invention is a device with easy installation and deinstallation.

The dosage of the drug and the duration of administration may be appropriately set depending on the degree of the control over the release from the device. For example, using the device of the invention, 0.01 to 10 g of the drug is administered over at least a month, preferably at least 3 months, further preferably at least 6 months, further preferably at least 1 year, further preferably at least 2 years, further preferably at least 3 years, further preferably at least 4 years, further preferably at least 5 years.

### 4. Drug

The disease to be treated with the controlled drug release device of the invention is not particularly limited but is a disease for which continual administration of a drug to the body is desired, particularly a disease for which local continual administration is desired. Such diseases are cancers, inflammatory diseases, degenerative diseases and the like.

Examples thereof include eye diseases, and a scleral placement device can be used for treating an eye disease. Examples of eye diseases include retinitis pigmentosa, age-related macular degeneration, glaucoma and the like, in which multiple factors such as genes and environmental factors are involved, retinal vascular disorders such as retinal artery occlusion, branch retinal vein occlusion and diabetic retinopathy and diseases causing inflammation or disorders in the choroid/retina/vitreous body such as uveitis. Retinitis pigmentosa is a disease with progressive damage in retinal neuron cells due to unknown causes and is designated to the incurable disease (specified disease). Retinitis pigmentosa is an eye disease with progressive degeneration of visual cells, and apoptosis (cell death) or the like of visual cells is caused due to various genetic abnormalities, inflammation, immune response or the like. Age-related macular degeneration is an eye disease with the appearance of neovessels and the like in the macular area with the age and is a specified disease in which neovessels are generated from the choroid located at the outside of the retina, resulting in the leakage of blood and a damage in the retina. Glaucoma is a progressive disease with characteristic optic disc change and abnormal visual field. A high intraocular pressure has been considered as the cause thereof, but many patients have been found to be suffering from glaucoma even though their intraocular pressures were in the normal range, and the weakness in the optic disc is also considered as a cause of glaucoma. The intraocular pressure, however, is the major risk factor for the progress of glaucoma, and the basic method for treating glaucoma is decreasing the intraocular pressure with a drug to stop the progress of the visual field defect. Recently, glaucoma has been the first cause of visual loss in Japan. In addition, subjects include treatment-resistant retinal artery occlusion, branch retinal vein occlusion, diabetic retinopathy, uveitis and the like. Controlled-release of a drug to an eye tissue is enabled by placing the device of the invention on the sclera, and the drug can be released particularly to the retina or choroid/retinal pigment epithelium (RPE).

The drugs used for treating a disease such as the eye diseases with the device of the invention are a drug which inhibits angiogenesis, a drug which promotes the growth of nerve cells, a drug which protects nerve cells, a steroid, an agent for treating glaucoma, an antiinflammatory agent, an antifungal agent, an anticancer agent, an immunosuppressive agent and the like. Moreover, isopropyl unoprostone (UNO) is used as an agent for treating retinitis pigmentosa, and the agent for inhibiting angiogenesis is vasohibin or the like. The agent for promoting the growth of nerve cells is BDNF (brain-derived neurotrophic factor) or the like, and the steroid is betamethasone, hydrocortisone or the like. As the agents for treating glaucoma, prostaglandin-related drugs (latanoprost, travoprost, tafluprost, unoprostone and the like), sympatholytic agents (timolol maleate, timolol gel, carteolol hydrochloride, betaxolol hydrochloride, levobunolol hydrochloride, nipradilol, bunazosin hydrochloride and the like), carbonic anhydrase inhibitors (dorzolamide hydrochloride, brinzolamide and the like) and the like, which are drugs mainly for lowering intraocular pressure, are mainly used. For a subcutaneously embedded device, in addition to the above drugs, a proangiogenic factor for forming vascular bed for creating space for cell transplantation is used. The proangiogenic factor is not particularly limited as long as the factor can induce angiogenesis, but examples thereof include basic fibroblast growth factor (bFGF), acidic fibroblast growth factor (aFGF), vascular endothelial growth factor (VEGF), platelet-derived growth factor, transforming growth factor-β (TGF-β), hepatocyte growth factor (HGF), osteonectin, angiopoietin and the like. Together with the proangiogenic factor, an immunosuppressive agent may be contained to avoid rejection in the case of allogeneic transplantation for cell transplantation after the formation of vascular bed and the space for cell transplantation. The immunosuppressive agent is cyclosporine, tacrolimus, sirolimus, a derivative thereof or the like. Insulin for controlling the blood glucose level is used as a bioactive substance (hormone).

The drug may be contained in the injectable gel of 1. or may contain another pharmaceutically acceptable carrier, excipient, lubricant, binder, disintegrating agent, coating agent or the like. The excipient is lactose, glucose, cornstarch, sorbitol, crystalline cellulose or the like, and the lubricant is talc, magnesium stearate, hardened vegetable oil or the like. The binder is dimethyl cellulose, polyvinyl alcohol, polyvinyl ether, methyl cellulose, ethyl cellulose, gum arabic, hydroxypropyl cellulose, polyvinylpyrrolidone or the like, and the disintegrating agent is starch, sodium alginate, gelatin powder, calcium carbonate, calcium citrate, dextrin or the like. When the drug is a liquid, the drug may contain a buffer, physiological saline or the like.

In the invention, the drug is also called a drug composition.

### Examples

Although the invention is specifically explained with Examples below, the invention is not limited by the Examples.

### [Example 1]

In this section, hydrogels which are not easily decomposed were placed in advance in devices, and use thereof was examined. A gel is cured in advance in a device, and then a drug solution is injected for drug release. Then, reinjection of the drug is achieved. After an *in vitro* drug release test using gels, use of the selected polyvinyl alcohol gel in the device was examined and assessed.

### 1 Reagents Used

Polydimethylsiloxane (PDMS, TORAY dow corning)
Agarose (Agarose-I, Dojindo)
PVA (poly(vinyl alcohol), Mw=~145000, SIGMA-ALDRICH)
AAm (Acrylamide, Mw=71.08, Wako)
MBAAm (N,N'-methylenebisacrylamide, Mw=154.17, Wako)
APS (Ammonium persulfate, Mw=228.2, Wako)
TEMED (N,N,N',N'-tetramethyl-ethylenediamine, Mw=116.2, Wako)
AMPS (2-Acrylamide-2-methylpropansulfonic acid, Mw=207.25, Wako)
2-Oxoglutaric Acid (Mw=146.1, Wako)
Fluorescein (free acid) (Mw=332.31, SIGMA-ALDRICH)
DPBS(-) (pH=7.4; 2.68 mM KCL, 1.47 mM KH₂PO₄, 136.9 mM NaCl, 8.06 mM Na₂HPO₄, Wako)

### 2 Method

In the experiment of this Example, a controlled-release test for assessing the controlled drug release properties of the produced devices with time was conducted. All the samples were measured using a fluorescence microplate reader (Gemini EM, Molecular Device), and the concentrations were determined from a calibration curve which was produced in advance. A 96-well black plate was used for the measurement, and the measurement was made using 200 µL of a sample solution.

### 2.1 Selection of Hydrogel

### 2.1.1 Production of Hydrogels

In this Example, hydrogels which are not easily decomposed were placed in devices, and drug release and reinjection were thus achieved. The details of the production procedures of the four kinds of hydrogel which were produced as candidates are shown.

### (1) Agarose Gel (AGA Gel)

(i) Distilled water was added to agarose powder to a concentration of 2 wt%.
(ii) The liquid of (i) was heated at 75°C while the liquid was mixed until the agarose powder was completely stirred, (iii) The sol produced in (ii) was poured into a dish and cooled (4°C), and an agarose gel was thus obtained. Then, the agarose gel was cut into an appropriate size and used.

### (2) Polyvinyl Alcohol Gel (PVA Gel)

(i) In a screw cap bottle, distilled water was added to PVA powder to a concentration of 15 wt%.
(ii) A beaker was filled with water, and the solution produced in (i) was placed in hot water. The environment for the water bath was the hotplate temperature of 120°C to 130°C and the speed of rotation of 50 rpm.
(iii) When the PVA in the solution was completely dissolved, the solution was poured into a dish and then frozen and thawed for gelation. For freezing and thawing, freezing (-30°C) for an hour and thawing (room temperature) for 10 to 15 minutes were repeated three times.

### (3) Polyacrylamide Gel (AAm Gel)

(i) AAm and MBAAm were added to 10 mL of distilled water to concentrations of 180 mg/mL and 0.118 mg/mL, respectively, and an aqueous monomer solution was thus produced.
(ii) A polymerization initiator (APS, 10 µL/10 mL) and a polymerization accelerator (TEMED, 100 µL/10 mL) were added to the aqueous solution produced in (i) and mixed thoroughly, and an AAm polymerization solution was thus produced.
(iii) The solution produced in (ii) was poured into a dish and left to stand still in an environment at 70°C, and an AAm gel was thus obtained.
(iv) The AAm gel produced in (iii) was washed with distilled water, and the unreacted chemical substances were removed completely from the inside of the gel. Then, the gel was cut into an appropriate size and used.

### (4) Double Network Gel (DN Gel)

(i) Deionized water was added to powder of AMPS (2.07 g, a monomer), MBAAm (61.6 mg, a crosslinking agent) and 2-oxogulutaric acid (3 mg, a photoinitiator) up to 10 mL, and an AMPS polymerization solution was thus obtained.
(ii) Deionized water was added to powder of AAm (2.84 g, a monomer), MBBAm (6.16 mg, a monomer) and 2-oxogulutaric acid (3 mg, a photoinitiator) up to 40 mL, and an AAm polymerization solution was thus obtained.
(iii) Dissolved oxygen was removed sufficiently by blowing nitrogen into the polymerization solution produced in (i) (10 min). Then, the solution was poured into a mold and irradiated with UV light (265 nm, 8W, 6h) in a nitrogen environment, and a PAMPS gel was thus obtained.
(iv) The PAMPS gel produced in (iii) was immersed in the PAAm monomer solution produced in (ii) for 24 hours (light shielded, 4°C). For the immersion, care should be devoted to a significant increase in the volume due to the absorption of the solution by the PAMPS gel.
(v) The PAMPS gel immersed in the AAm monomer solution for 24 hours was placed between glass plates and irradiated with UV light in a nitrogen environment, and a DN gel was thus obtained.
(vi) The DN gel produced in (v) was washed with distilled water, and the unreacted chemical substances were removed completely from the inside of the gel.

### 2.1.2 In Vitro Drug Release of Hydrogels

An *in vitro* drug release test using the four kinds of hydrogel produced in 2.1 was conducted to determine the gel suitable for introduction into the device. First, the produced gels were cut into a size. Next, a fluorescein solution (50 mg/mL) was dropped onto the gels, and the gels were permeated with fluorescein. After an overnight process, the gels were placed in 10 mL of DPBS(-), and the fluorescence intensities were measured using a plate reader. Here, the measurement was made every one to three days until the fluorescence intensities became the same as those of DPBS.

### 2.2 In Vitro Drug Release from PVA Gel-Introduced Device and Reinjection of Drug into Device

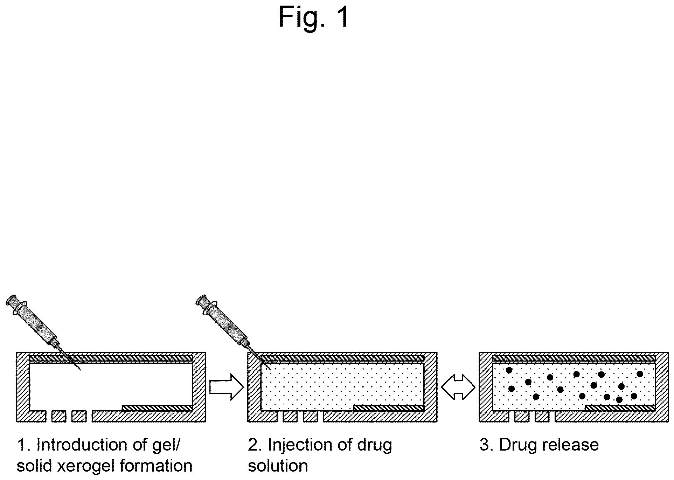

As described below, the gel which was most suitable for introduction into the device was the PVA gel from the results of 2.1.2. As a mechanism for drug release/reinjection using the PVA gel, the mechanism illustrated in Fig. 1 was produced. In the mechanism, after curing the PVA gel and forming a xerogel in the device, a drug solution was injected, and the gel was permeated with the solution. Then, the drug was released, and the drug solution was injected again into the device, thereby achieving reinjection of the drug and drug release.

### 2.2.1 Scanning Electron Microscope Observation

To observe the mesh structure of the PVA gel, the PVA gel was observed using a scanning electron microscope (KEYENCE, VE-9800). For the observation, a PVA gel sample was produced, and the sample was made conductive by coating with Ti·Pt·Au by sputtering using a sputtering system (SANYUELECTRON, SVC-700TM SG) in advance.

### 2.2.2 Drug Release from PDMS/PVA Device

An aqueous PVA solution was injected into a PDMS capsule having no incorporated stainless-steel sheet/stainless-steel wire and then subjected to repeated freezing and thawing to fill the capsule with the aqueous PVA solution and form a gel. When a xerogel was formed, an aqueous solution containing a drug was injected. As the aqueous model drug solution, an aqueous fluorescein (FL) solution (50 mg/mL) was used. The capsule was placed in 10 mL of DPBS(-) for drug release. The solvent was changed every one to three days, and the fluorescence intensity was measured each time. The end of the test was the point where the fluorescence intensity value became equivalent to the value of the solvent. As a control, the fluorescence intensities of a PDMS capsule into which the aqueous FL solution was injected were also measured in the same manner.

### 2.2.3 Drug Release from PDMS/PVA Device Replenished with Aqueous FL Solution

The PDMS capsule after the completion of the drug release in 2.2.2 was filled again with the aqueous FL solution by injecting into the PDMS capsule using a 1 mL syringe and a 25G injection needle. Through this procedure, "2. Injection of Drug Solution" was conducted again for the capsule which had already completed "3. Drug Release" once in Fig. 1. The capsule was placed in 10 mL of DPBS(-) for drug release. The solvent was changed every one to three days, and the fluorescence intensity was measured each time. The end of the test was the point where the fluorescence intensity value became equivalent to the value of the solvent.

### 2.3 In Vitro Drug Release from Partial PVA Gel-Introduced Device and Reinjection of Drug into Device

As a mechanism for drug release/reinjection using a PVA gel, the mechanism illustrated in Fig. 2 was developed. In this mechanism, after partially curing a PVA gel in the device only at the pore side, a drug solution is injected from the bottom of the device, and the solution is thus introduced. Then, the drug is released while the PVA gel functions like an osmosis membrane for the drug. When the drug solution is injected again into the device, the liquid gathered in the device is suctioned, and then the drug solution is injected to achieve reinjection of the drug and drug release.

In the same manner as in 2.2.2, the aqueous PVA solution was injected into a PDMS capsule having no incorporated stainless-steel sheet/stainless-steel wire and then subjected to repeated freezing and thawing, and the aqueous PVA solution was formed into a gel in the capsule. At this point, the PVA gel was introduced only at the pore side as illustrated in Fig. 2, and the aqueous FL solution was injected into the lower part of the capsule. The capsule was placed in 5 mL of DPBS(-) for drug release. The solvent was changed every one to three days, and the fluorescence intensity was measured each time. The end of the test was the point where the fluorescence intensity value became equivalent to the value of the solvent.

### 2.4 Statistical Analysis

The data described are all average values ± standard deviations. For the comparison between two groups, Student' s-T test was used for significant difference test. Excel was used, and it was determined that there was a significant difference in the case of p < 0.05.

### 3 Results and Discussion

### 3.1 In Vitro Drug Release of Hydrogels

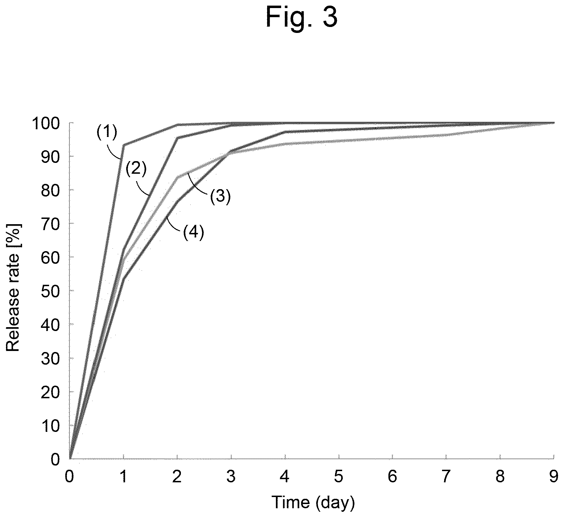

In accordance with the procedures in 2.1.1, four kinds of gel, namely AGA, PVA, AAm and DN, were successfully produced. The results of the drug release test using the gels conducted by the procedures in 2.1.2 are shown in Fig. 3. The AGA gel shown with (1) released most of the drug in one day. The DN gel shown with (2) released most of the drug in around two days. The remaining two kinds of gel ((3) AAm gel and (4) PVA gel) exhibited similar release profiles, but the swelling of the AAm gel was significant. Considering the swelling and the biocompatibility of acrylamide, the AAm gel is not suitable for use for introduction into a PDMS capsule. Therefore, the PVA gel, which releases the drug slowly and which is biocompatible with little swelling, was believed to be suitable for use in a PDMS capsule.

### 3.2 Scanning Electron Microscope Observation

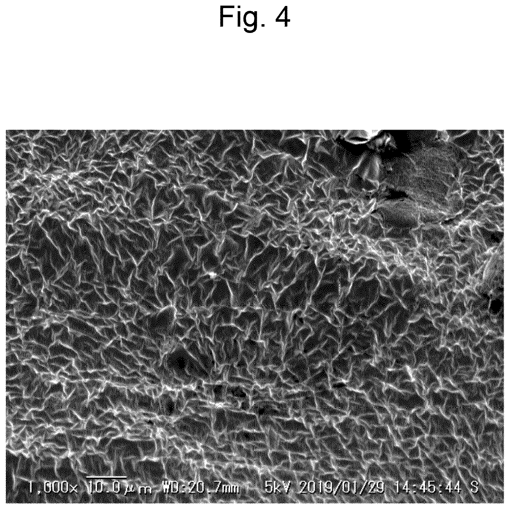

A SEM image of the PVA gel is shown in Fig. 4. The image shows that the PVA gel has a mesh structure having pores of around several micrometers. In general, a drug, even a high polymer drug, has a size of the angstrom order, and the drug is believed to permeate through the PVA gel.

### 3.3 Drug Release from PDMS/PVA Device

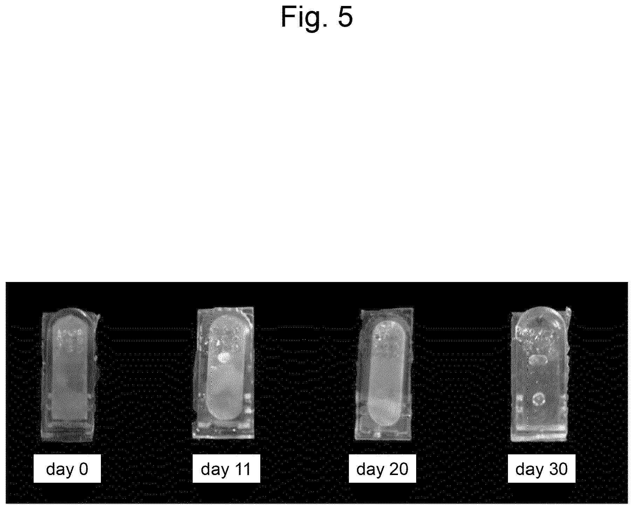

To achieve the mechanism of Fig. 1, the change of the device with time during the first drug release from the PDMS capsule conducted by the procedures of 2.2.2 is shown in Fig. 5. It can be seen that, as the drug was released from the pores of the capsule, which was filled with FL on day 0, the amount of FL in the device reduced on day 11 and day 20 (in the pictures in Fig. 5, the lines in the middle to the bottom of the device in the pictures of day 11 and day 20 are the boundary of FL) and that most of FL was released until day 30.

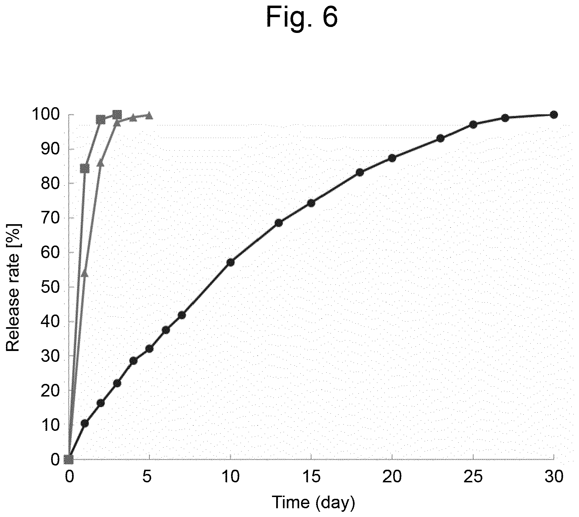

The results of the first drug release from the PDMS capsule are shown in Fig. 6 as the change in the release rate based on the final total release amount of FL. The release from the PVA gel alone shown in 2.1 is also shown. The release from the device produced by injecting the aqueous FL solution into the PDMS capsule, which is indicated with the black squares, almost completed in four days. The drug release from the PVA gel alone, which is indicated with black triangles, showed significant initial burst, and release of almost 100% was observed in around five days. On the other hand, controlled-release of FL over around four weeks was observed from the combination of the PDMS capsule and the PVA gel, which is indicated with black circles. This suggests that controlled-release of a drug can be achieved when the PDMS capsule and the PVA gel are combined.

### 3.4 Drug Release from PDMS/PVA Device Replenished with Aqueous FL Solution

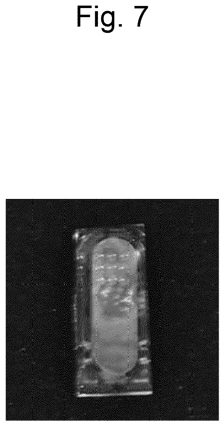

The capsule after the completion of reinjection of the aqueous FL solution is shown in Fig. 7. The reinjection of the drug and the filled capsule were observed.

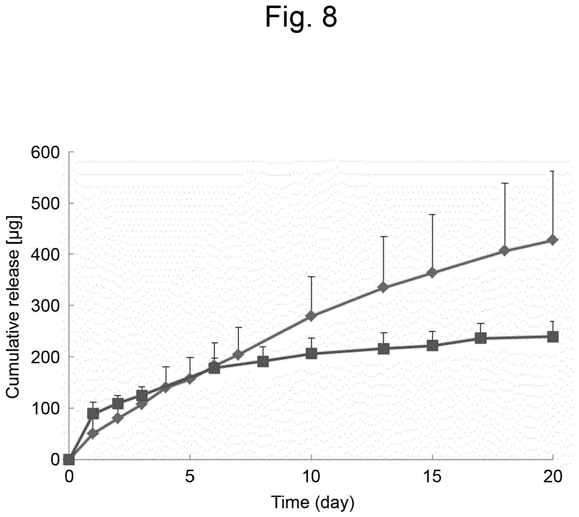

The results of the drug release amount of the capsule replenished with the drug are shown in Fig. 8 together with the results of the first drug release amount shown in 3.3. It can be seen that the second drug release, which is indicated with black squares, completed in around 10 days. This is believed to be because the amount of the introduced drug was lower than that of the first injection since the drug was introduced to the PVA gel that was swollen to some extent during the reinjection of the drug. Thus, a method of using another PVA gel is believed to be necessary for combination with the PDMS capsule.

### 3.5 Drug Release from PDMS/Partial PVA Device

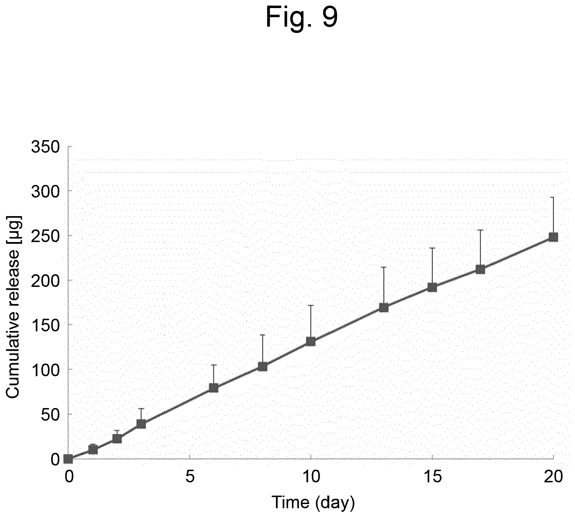

The results of the first drug release test of the PDMS capsule conducted to achieve the mechanism of Fig. 2 by the procedures in 2.3 are shown in Fig. 9. As a result, linear drug release was observed. Release exceeding 10 µg/day was observed from the capsule for three weeks. The amount exceeded the effective release amounts of low-molecular-weight drugs using transscleral DDS which have been confirmed so far, namely rabbit (10 µg/day) and monkey (12 µg/day) (Nagai N. et al., Investigative Ophthalmology & Visual Science, Vol.57, 6527-6538(2016); Nagai N. et al., Investigative Ophthalmology & Visual Science, Vol.59, 644-652(2018)). This suggests the *in vivo* effectiveness of the device. Moreover, because suction of the drug from the capsule and reinjection of the same amount of the drug as that of the first injection were possible, it was suggested that drug release and reinjection of the drug are enabled using the mechanism.

### 4 Summary

In this Example, a polyvinyl alcohol gel was introduced into a PDMS capsule, and the drug release and the potential for reinjection of the drug were examined. Drug release was observed from the drug-containing polyvinyl alcohol gel through release in DPBS. When the gel was incorporated into the entire PDMS capsule, while excellent drug release was observed during the first release after xerogel formation and subsequent injection of a drug solution, the amount of the second drug release after reinjection of the drug was lower than that of the first drug release. When the polyvinyl alcohol gel was incorporated into the half at the pore side, linear drug release was observed in the first release. Moreover, because suction of the liquid in the lower part of the capsule and reinjection of the drug were possible, the second drug release is believed to be equivalent to the first release. This means that incorporation of the polyvinyl alcohol gel in the PDMS capsule enabled drug release and reinjection of the drug.

### [Example 2] TEGDM Capsule Produced With 3D Printer

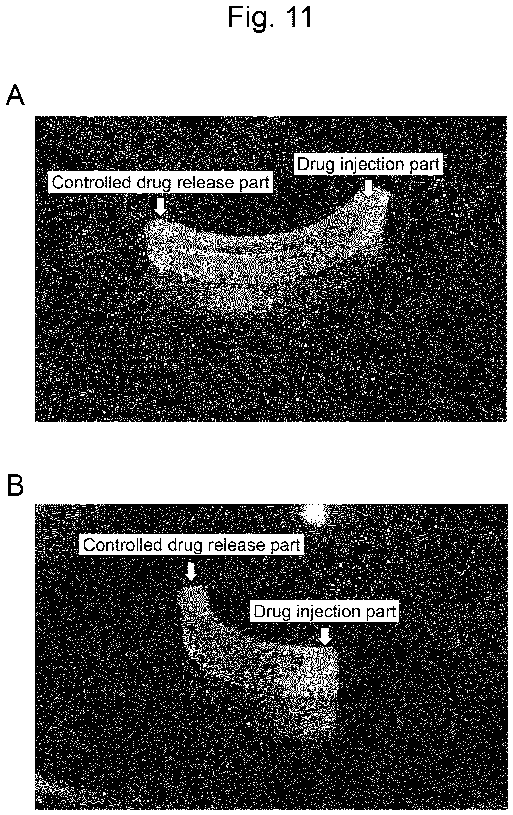

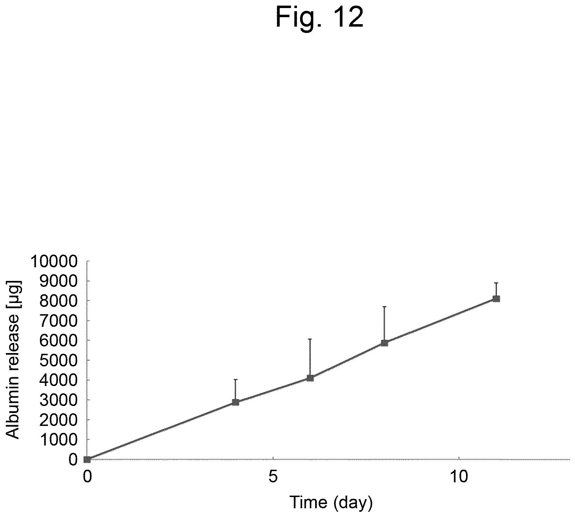

Using a 3D printer of stereo lithography type, a TEGDM capsule having space in the medicament introduction part and pores in the controlled medicament release part was produced. The structure is shown in Figs. 10 and 11. The structure itself is the same as that of the device of Embodiment 2. Fig. 10 shows the positional relationship of the controlled drug release part, the drug storage part and the drug introduction part of the device. A is the top view, and B is the side view. C shows how the drug is injected from the drug introduction part using a syringe and a needle. Fig. 11 shows the controlled drug release part and the drug introduction part of the device. PDMS was introduced into the drug introduction part, and a PVA gel was introduced into the controlled drug release part. The number of freeze-thaw cycles for the PVA gel was three. An aqueous FITC-albumin solution (250 mg/mL) was introduced into the finished capsule, and the capsule was placed in 5 mL of DPBS(-) for drug release. The solvent was changed every one to five days, and the fluorescence intensity was measured each time. As a result, linear release without any initial burst was observed (Fig. 12).

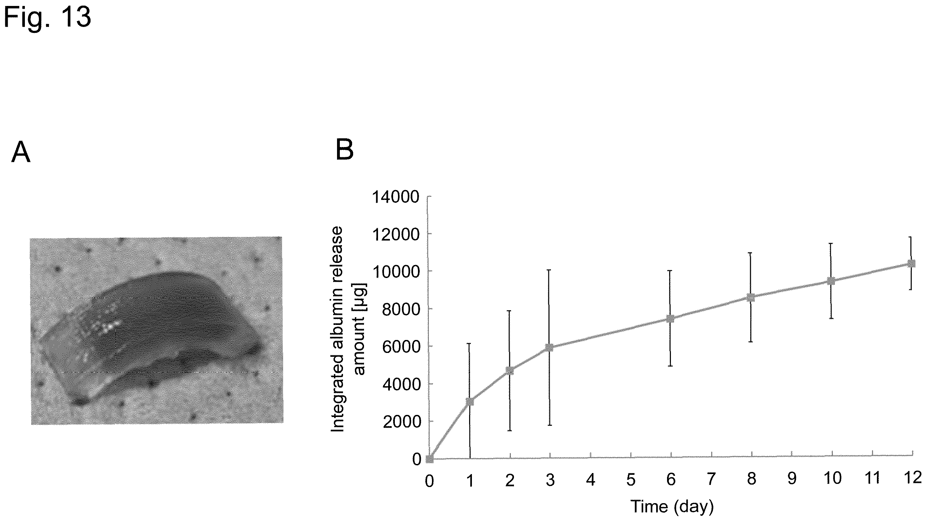

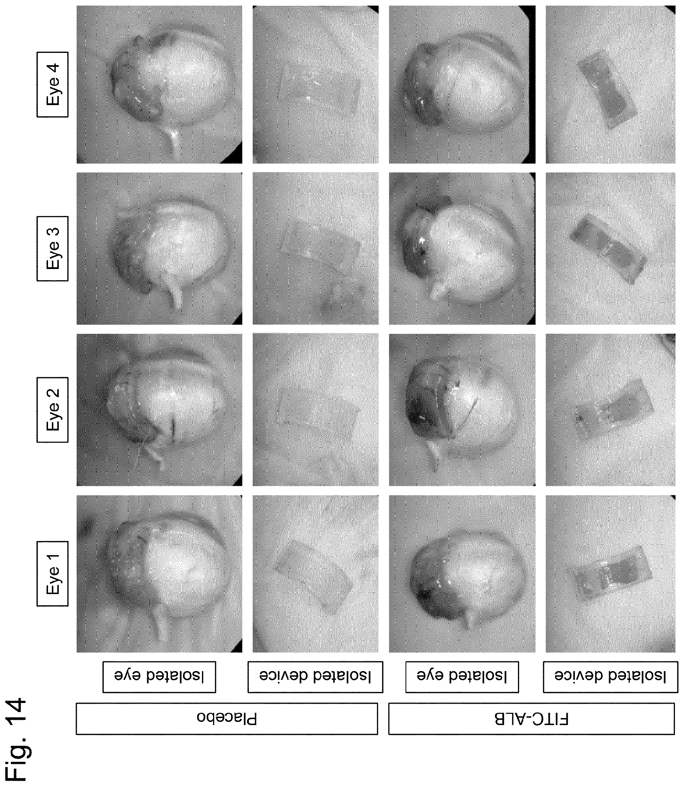

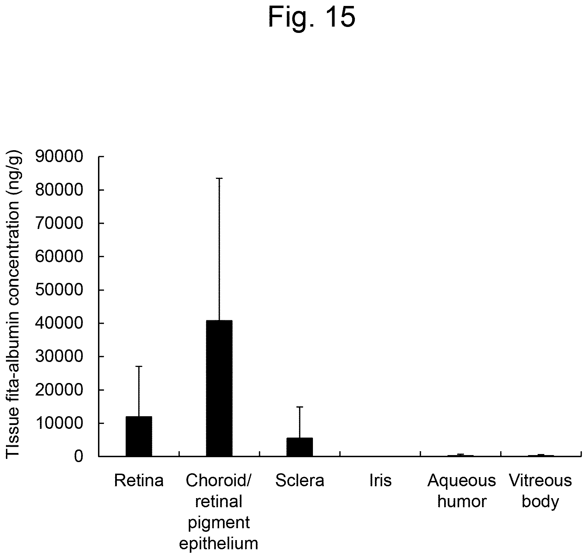

Using a 3D printer of stereo lithography type, a TEGDM capsule having space in the drug introduction part and pores in the controlled drug release part was produced. The size was adjusted to fit the rabbit eyes (length of 12 mm, width of 5,4 mm, thickness of 1.2 mm and diameter of curvature of 12 mm) because the device was for implantation in rabbits. PDMS was introduced into the drug introduction part, and a PVA gel was introduced into the controlled drug release part. The number of freeze-thaw cycles for the PVA gel was three. An aqueous FITC-albumin solution (250 mg/mL) was introduced into the finished capsule (Fig. 13A), and the capsule was placed in 5 mL of DPBS(-) for drug release. The solvent was changed every one to seven days, and the fluorescence intensity was measured each time. As a result, linear release without any initial burst was observed (Fig. 13B). FITC-albumin-containing capsules of this type were placed on the sclera of rabbits' eyes, and the eyeballs were taken out after two weeks and separated into retina, choroid/retinal pigment epithelium (RPE), sclera, iris, vitreous body and aqueous humor fractions. Homogenates thereof were prepared, and the fluorescence intensities were measured. As a control, placebo devices were prepared. The sample numbers were each N=4. The fluorescence intensities were converted into concentrations using a FITC-albumin calibration curve of known concentrations, and the concentrations were divided by the weights of the respective tissues and assessed as the tissue FITC-albumin amounts. The eyeballs and the devices taken out are shown in Fig. 14. The results of the FITC-ALB concentrations of the respective tissues are shown in Fig. 15. As a result, FITC-albumin was detected from the retina and the choroid/retinal pigment epithelium (RPE).

### Industrial Applicability

The replenishable continual drug release device of the invention can be used for continual administration of a drug over a long period.

The publications, the patents and the patent applications cited in the present specification are all incorporated directly in the present specification by reference.

## Claims

1. A controlled drug release device for implantation in a body that can be replenished with a drug using a syringe and a needle in the state of being implanted in the body,
wherein the device has a capsule structure and includes
(i) a controlled drug release part for controlled-release of a drug from the device,
(ii) a drug storage part for keeping the drug, and
(iii) a drug introduction part for introducing the drug into the drug storage part,
wherein the controlled drug release part has a controlled-release face having a pore,
a hydrogel is retained in the controlled drug release part,
the drug storage part is adjacent to the controlled drug release part,
the drug kept in the drug storage part is released from the pore in the controlled drug release part through the controlled drug release part, and
the drug is introduced into the drug storage part through the drug introduction part using a syringe and a needle.

2. The controlled drug release device that can be replenished with a drug according to claim 1, wherein the device having a capsule structure is made of polydimethylsiloxane (PDMS).

3. The controlled drug release device that can be replenished with a drug according to claim 1, wherein the device having a capsule structure is made of triethylene glycol dimethacrylate (TEGDM) and/or polyethylene glycol dimethacrylate (PEGDM) and has a layer of polydimethylsiloxane (PDMS) or urethane in the drug introduction part.

4. The controlled drug release device that can be replenished with a drug according to claim 1, wherein the device having a capsule structure is made of triethylene glycol diacrylate (TEGDA) and/or polyethylene glycol diacrylate (PEGDA) and has a layer of polydimethylsiloxane (PDMS) or urethane in the drug introduction part.

5. The controlled drug release device that can be replenished with a drug according to claim 1 or 2, wherein the hydrogel retained in the controlled drug release part is an agarose gel (AGA gel), a polyvinyl alcohol gel (PVA gel), a polyacrylamide gel (AAm gel) or a double network gel (DN gel).

6. The controlled drug release device that can be replenished with a drug according to any one of claims 1 to 4, wherein the hydrogel retained in the controlled drug release part is a polyvinyl alcohol gel (PVA gel), a mixture of polyethylene glycol dimethacrylate (PEGDM) and triethylene glycol dimethacrylate (TEGDM), a mixture of polyethylene glycol diacrylate (PEGDA) and triethylene glycol diacrylate (TEGDA) or polydimethylsiloxane having a porous structure (porous PDMS).

7. The controlled drug release device that can be replenished with a drug according to any one of claims 1 to 6 which can keep a liquid as the drug in the drug storage part.

8. The controlled drug release device that can be replenished with a drug according to any one of claims 1 to 7 which has an incorporated metal wire for changing and stabilizing the shape.

9. The controlled drug release device that can be replenished with a drug according to any one of claims 1 to 8 which is a scleral placement device for implantation in a sclera.

10. The controlled drug release device that can be replenished with a drug according to any one of claims 1 to 8 which is a subcutaneously embedded device for subcutaneous embedding.
